# EUROPEAN PATENT APPLICATION

(11) **EP 4 385 436 A1**
(43) Date of publication of application: **19.06.2024**
(21) Application number: 22855282.4
(22) Date of filing: 02.08.2022
(51) Int. Cl.: A61B 18/02

(54) **MULTI-CHANNEL CRYOABLATION SYSTEM AND CONTROL METHOD**

(30) Priority: 12.08.2021 CN 202110923061
(71) Applicant: Accu Target Medipharma (Shanghai) Co., Ltd., Shanghai 200000 (CN)
(72) Inventor: YU, Weiqiu, Shanghai 201318 (CN); XU, Binkai, Shanghai 201318 (CN); CHANG, Zhaohua, Shanghai 201318 (CN)
(74) Representative: Argyma
(86) International application number: PCT/CN2022/109746
(87) International publication number: WO 2023/016299

(57) **Abstract**

Disclosed is a multichannel cryoablation system, which employs a high-pressure nitrogen as a gas source. The system includes a main gas pipeline, a rewarming pipeline, a refrigeration high-pressure pipeline, a refrigeration low-pressure pipeline, and N channel pipelines. The main gas pipeline is provided with a gas source input port, the rewarming pipeline is communicated with the main gas pipeline, and the rewarming pipeline is divided into N rewarming branch channels. The refrigeration high-pressure pipeline is communicated with the main gas pipeline, and the refrigeration low-pressure pipeline is communicated with the main gas pipeline, and the refrigeration low-pressure pipeline shares a section of gas supply path with a rear section of the refrigeration high-pressure pipeline, the gas supply path being communicated with N refrigeration branch channels. Each of N channel pipelines has one end used to be connected with an ablation needle, and the other end used to be connected with one of the refrigeration branch channels and/or one of the rewarming branch channels, wherein N is a positive integer greater than or equal to 2.

## Description

### BACKGROUND OF THE INVENTION

### Field of the Invention

The invention relates to a multichannel cryoablation system employing high-pressure nitrogen as a gas source, and a multichannel cryoablation control method implemented on the system.

### Description of the Prior Art

As a minimally invasive targeted surgery, cryoablation has the characteristics of less trauma, less toxic and side effects, and definite curative effect, as well as the advantages of clear boundary of ablation ice puck, being capable of participating in the activation of tumor immune function of the body, no damage on large blood vessels, and no obvious pain. This makes ultra-low temperature targeted cryotherapy and hyperthermia of tumors a reality. In recent years, cryosurgery has been widely used in the treatment of metastatic liver cancer, prostate cancer, kidney cancer, etc.

The multichannel cryoablation system has the function of combining multiple ablation needles. Since the frozen area formed by a single ablation needle in human tumor tissue is limited, the combined use of multiple ablation needles can expand the frozen area and effectively cover larger tumor tissue when the volume of tumor tissue is large. The cryoablation system is designed to have multiple channels, which can be connected to multiple ablation needles, so that through the combination of the number of ablation needles, different volumes of tumor tissue can be ablated, making the applicability of the device stronger.

There are two mainstream multichannel cryoablation systems currently on the market:
Argon Helium Cryogenic System is described in related patent documents such as CN208756146U, which discloses a cryogenic surgery system that uses 3000 psi argon as a cryogenic gas source.
Liquid Nitrogen Cryoablation System is described in related patent documents such as CN210582629U, which discloses a cryoablation system that uses cryogenic liquid nitrogen as a refrigeration source.

At present, there is no multichannel cryoablation device or system that uses high-pressure nitrogen as a gas source.

### SUMMARY OF THE INVENTION

The invention provides a multichannel cryoablation system and a control method using the same, which are suitable for employing high-pressure nitrogen as a gas source.

The technical solution of the invention is as follows:
A multichannel cryoablation system employs high-pressure nitrogen as a gas source, and includes:
a main gas pipeline, provided with a gas source input port, a first pressure measuring device, and a main gas valve, wherein the gas source input port is used to be connected with a high-pressure nitrogen source, the first pressure measuring device is used to obtain a gas pressure of the high-pressure nitrogen cylinder, and the main gas valve may realize the on/off of the high-pressure nitrogen in the main gas pipeline;
a rewarming pipeline, communicated with the main gas pipeline; the rewarming pipeline is provided with a first gas output pressure regulating device, a second pressure measuring device, and a rewarming pipeline main valve, wherein the first gas output pressure regulating device may regulate a gas output pressure of the rewarming pipeline, the second pressure measuring device is used to obtain an output pressure of the first gas output pressure regulating device, and the rewarming pipeline main valve may realize the on/off of the high-pressure nitrogen in the rewarming pipeline; the rewarming pipeline is divided into N rewarming branch channels following the rewarming pipeline main valve, and each of the rewarming branch channels is provided with a branch channel valve to realize the on/off of a gas circuit;
a refrigeration high-pressure pipeline and a refrigeration low-pressure pipeline, communicated with the main gas pipeline respectively, wherein the refrigeration high-pressure pipeline is provided with a second gas output pressure regulating device, a third pressure measuring device and a refrigeration high-pressure pipeline main valve; the second gas output pressure regulating device may regulate a gas output pressure of the refrigeration high-pressure pipeline, the third pressure measuring device is used to obtain an output pressure of the second gas output pressure regulating device, and the refrigeration high-pressure pipeline main valve may realize the on/off the high-pressure nitrogen in the refrigeration high-pressure pipeline; the refrigeration low-pressure pipeline is provided with a third gas output pressure regulating device, a fourth pressure measuring device and a refrigeration low-pressure pipeline main valve; the third gas output pressure regulating device may regulate a gas output pressure of the refrigeration low-pressure pipeline, the fourth pressure measuring device is used to obtain an output pressure of the third gas output pressure regulating device, and the refrigeration low-pressure pipeline main valve may realize the on/off of the high-pressure nitrogen in the refrigeration low-pressure pipeline; and a part of the refrigeration low-pressure pipeline following the refrigeration low-pressure pipeline main valve shares a section of gas path with a part of the refrigeration high-pressure pipeline following the refrigeration high-pressure pipeline main valve, and the gas path is communicated with the N refrigeration branch channels, each of the refrigeration branch channels being provided with a branch channel valve to realize the on/off of the gas circuit;
N channel pipelines, each of the channel pipelines having one end used to be connected with an ablation needle, and the other end used to be connected with one of the refrigeration branch channels and/or one of the rewarming branch channels; each of the channel pipelines is further provided with a fifth pressure measuring device, a pipe exhaust hole and a valve for controlling the opening and closing of the pipe exhaust hole; each of the ablation needles is connected with an air outlet or is provided with the air outlet;
   wherein N is a positive integer greater than or equal to 2. When N is a positive integer equal to 2, the multichannel cryoablation system is a two-channel cryoablation system. And so on

In a preferred embodiment, N is a positive integer greater than or equal to 3. When N is a positive integer equal to 3, the multichannel cryoablation system is a three-channel cryoablation system. And so on

In a preferred embodiment, the main gas valve, the rewarming pipeline main valve, the refrigeration high-pressure pipeline main valve and the refrigeration low-pressure pipeline main valve are electromagnetic valves, the branch channel valve is the electromagnetic valve, and the valve for controlling the opening and closing of the pipe exhaust hole is the electromagnetic valve. The electromagnetic valves are selected for automatic control via the control module.

In a preferred embodiment, the first/second/third gas output pressure regulating device is a pressure reducing valve. The pressure reducing valve may be a manually-adjusted pressure reducing valve or a pressure reducing valve automatically controlled by the control module.

In a preferred embodiment, the ablation needle is further provided with an electrical interface, and the electrical interface includes a temperature measurement device wire, a rewarming thermal resistance wire and an identification interface wire; the multichannel cryoablation system further includes a control module, and the control module includes a pressure measuring module, a temperature measuring module, a switch module, an ablation needle locking module and an ablation needle identification module, wherein the control module obtains all data of the pressure measuring device through the pressure measuring module, obtains all temperatures of the ablation needle through the temperature measuring module, controls switching of the electromagnetic valve and a rewarming power supply of the ablation needle through the switch module, locks a gas interface between the ablation needle and the channel pipeline through the ablation needle locking module, and identifies the ablation needle through the ablation needle identification module. Such arrangement makes the multichannel cryoablation system highly automatic.

Based on the same inventive idea, the invention further provides a multichannel cryoablation control method, which is capable of being implemented in the multichannel cryoablation system according to any one of the above items, and includes:
preparation of the ablation needle: marking each of the ablation needles as "prepared" or "unprepared" in the control module, wherein marking the ablation needle as "prepared" needs to meet the conditions that an ablation needle locking interface is locked, the temperature of the ablation needle may be collected, the rewarming power supply is available, and the ablation needle identification interface is available, and if any of the above conditions are not met, the corresponding ablation needle is marked as "unprepared";
selection of the refrigeration high-pressure pipeline and the refrigeration low-pressure pipeline: determining to select the refrigeration high-pressure pipeline and the refrigeration low-pressure pipeline according to the number of the ablation needles prepared in the stage of the preparation of the ablation needle, wherein when more than three ablation needles are used, the refrigeration high-pressure pipeline is selected, and when less than three ablation needles are used, the refrigeration low-pressure pipeline is selected;
use of the ablation needle: cryoablating and rewarming the selected one or more ablation needles, wherein when the on/off of the gas path is controlled at some stage in the process of cryoablating and rewarming each of the ablation needles, the opening and closing is required to be performed for the main gas pipeline, the rewarming pipeline main valve, the refrigeration high-pressure pipeline main valve or the refrigeration low-pressure pipeline main valve according to requirements for the gas path at the stage of treatment using the remaining ablation needles of the shared pipeline.

In a preferred embodiment, the control method further includes an exhaust method of exhausting the gas before unlocking an ablation needle gas interface.

In a preferred embodiment, the control method further includes performing one of the exhausting operations after a refrigerating function or a rewarming function of the ablation needle is completed.

In a preferred embodiment, the control method further includes sending a prompt signal when the first pressure measuring device detects that a pressure value of the gas cylinder is lower than a preset lower limit pressure value.

In a preferred embodiment, an output pressure of the first/second/third gas output pressure regulating device is regulated manually, the control module determines whether the output pressure of the first/second/third gas output pressure regulating device is within a predetermined range according to the pressure value when the first/second/third gas output pressure regulating device is regulated to further judge failures of the first/second/third gas output pressure regulating device.

In a preferred embodiment, the control method further includes determining whether the gas reaches the place, by the control module, according to a pressure value measured by the fifth pressure measuring device on each of the channel pipelines.

In a preferred embodiment, the control method further includes determining that a gas in an exhaust pipe on the channel pipeline has been exhausted when an exhausting function of the ablation needle is performed such as the control module receiving information that the pressure value measured by the fifth pressure measuring device on the channel pipeline is close to 0.

Based on the same inventive idea, the invention further provides a multichannel cryoablation system, which employs high-pressure nitrogen as a gas source, and includes:
a main gas pipeline, provided with a gas source input port, the gas source input port being used to be connected with the high-pressure nitrogen source; the main gas pipeline is connected and communicated with at least one rewarming pipeline and at least one refrigeration pipeline respectively, wherein
each of the rewarming pipelines is provided with a first gas output pressure regulating device, and the first gas output pressure regulating device may regulate gas output pressure of the corresponding rewarding pipeline; each of the rewarming pipelines is divided into N rewarming branch channels following the first gas output pressure regulating device, each of the rewarming branch channels is provided with a branch channel valve, and the branch channel valve may realize the on/off of the high-pressure nitrogen in the rewarming branch channel;
each of the refrigeration pipelines is provided with a second gas output pressure regulating device, and the second gas output pressure regulating device may regulate the gas output pressure of the refrigeration pipeline; each of the refrigeration pipelines is divided into N refrigeration branch channels following the second gas output pressure regulating device, each of the refrigeration branch channels is provided with a branch channel valve, and the branch channel valve may realize the on/off of the high-pressure nitrogen in the refrigeration branch channel;
N channel pipelines, each of the channel pipelines having one end used to be connected with an ablation needle, and the other end used to be connected with one of the refrigeration branch channels and/or one of the rewarming branch channels;
   wherein N is a positive integer greater than or equal to 2.

For the multichannel cryoablation system of the invention, the number of refrigeration pipelines may be one, two, three, or more. The use of one or more refrigeration pipelines is determined according to the number of designed channels of the system and the number of actually-used channels; for example, if the multichannel cryoablation system is equipped with three channels, it is necessary to distinguish between high-pressure and low-pressure pipelines, and if the multichannel cryoablation system is only designed with two channels, then the pipeline only has low-pressure pipelines and no high-pressure pipelines; if three channels are designed and only two of them are used in actual use, only low-pressure pipelines are required; while three channels are actually used, then the high-pressure pipelines are required. If there are enough channels, such as more than three channels, it may be necessary to arrange refrigeration high-pressure/medium-pressure/low-pressure pipelines.

In a preferred embodiment, the multichannel cryoablation system includes more than two refrigeration pipelines, wherein pressures in the refrigeration pipelines are different from each other. In the structure of two refrigeration pipelines, the gas pressures in the refrigeration pipelines are different, which may be called the refrigeration high-pressure pipeline and the refrigeration low-pressure pipeline; in the structure of three refrigeration pipelines, the gas pressures in the refrigeration pipelines are different, which may be called the refrigeration high-pressure pipeline, a refrigeration medium-pressure pipeline, and the refrigeration low-pressure pipeline.

For the multichannel cryoablation system with more than two refrigeration pipelines, each of the refrigeration pipelines may be provided with a refrigeration branch channel respectively; however, more preferably, the more than two refrigeration pipelines may be designed as a multiplexing structure, at least two refrigeration pipelines share a section pipeline following the respective second gas output pressure regulating device, and the shared section of pipeline may be communicated with each of the refrigeration branch channels. The more than two refrigeration pipelines are designed as the multiplexing structure, which may greatly save the number of refrigeration branch channels, and reduce the cost and complexity of equipment installation.

In a preferred embodiment, each of the rewarming pipelines is provided with a rewarming pipeline main valve, and the rewarming pipeline main valve may realize the on/off of the high-pressure nitrogen in the rewarming pipeline; each of the refrigeration pipelines is provided with a refrigeration pipeline main valve, and the refrigeration pipeline main valve may realize the on/off of the high-pressure nitrogen in the refrigeration pipeline. The provisions of the main valves on each of the above pipelines may make controlling the gas path on each of the pipelines more reliable.

In a preferred embodiment, the main gas pipeline is provided with a main gas valve, and the main gas valve may realize the on/off of the high-pressure nitrogen in the main gas pipeline. The provision of the main gas valve may make controlling the gas path on the main gas pipeline more reliable.

In a preferred embodiment, the system further includes the first pressure measuring device provided on the main gas pipeline, wherein the first pressure measuring device is used to measure a gas pressure of the high-pressure nitrogen cylinder. The provision of the first pressure measuring device may judge whether the gas source is available according to pressure measurement results thereof and a preset lower limit value.

In a preferred embodiment, each of the rewarming pipelines is provided with a second pressure measuring device, and the second pressure measuring device is used to obtain an output pressure of the first gas output pressure regulating device; each of the refrigeration pipelines is provided with a third pressure measuring device, and the third pressure measuring device is used to obtain an output pressure of the second gas output pressure regulating device. The provisions of the above pressure measuring devices may determine whether the output pressure of the corresponding pressure reducing valve is within a predetermined range according to the respective measured pressure values, and then determine whether the pressure reducing valve is faulty.

In a preferred embodiment, each of the channel pipelines is further provided with a pressure measuring device. In this way, the following situations may be realized: when the freezing and rewarming functions of the ablation needle are performed, the pressure values measured by the pressure measuring device may judge whether the gas on the corresponding channel pipeline reaches here, wherein if the pipeline between the channel pipeline and the gas source is blocked, the gas may not reach here. In addition, when the exhaust operation is performed, it can be known that the gas in the exhaust pipe has been exhausted when the pressure value measured by the pressure measuring device is close to 0.

The above pressure measuring devices may be pressure sensors.

In a preferred embodiment, each of the channel pipelines is further provided with a pipe exhaust hole, and a valve for controlling the opening and closing of the pipe exhaust hole; each of the ablation needles is connected with an air outlet or is provided with the air outlet. This arrangement realizes gas exhaust of the channel pipelines and the ablation needle.

In a preferred embodiment, all valves used in the pipelines to perform the on-off are electromagnetic valves. The electromagnetic valves are selected for automatic control via the control module.

In a preferred embodiment, the first/second gas output pressure regulating device is a pressure reducing valve. The pressure reducing valve may be a manually-adjusted pressure reducing valve or a pressure reducing valve automatically controlled by the control module.

In a preferred embodiment, the ablation needle is provided with an electrical interface, and the electrical interface includes a temperature measurement device wire, a rewarming thermal resistance wire and an identification interface wire; the multichannel cryoablation system further includes a control module, and the control module includes a pressure measuring module, a temperature measuring module, a switch module, an ablation needle locking module and an ablation needle identification module, wherein the control module obtains all data of the pressure measuring device through the pressure measuring module, obtains all temperatures of the ablation needle through the temperature measuring module, controls switching of the electromagnetic valve and a rewarming power supply of the ablation needle through the switch module, locks a gas interface between the ablation needle and the channel pipeline through the ablation needle locking module, and identifies the ablation needle through the ablation needle identification module.

Above, the symbol "/" means "and/or", "and", or "or".

Compared with conventional art, the invention has the following beneficial effects:
The invention first provides a multichannel cryoablation system employing a high-pressure nitrogen as a gas source, and a control method therefor, so that the use of high-pressure nitrogen in the multichannel system becomes possible, and the advantages of high-pressure nitrogen, high cooling capacity, and cheaper and easier access to gas sources may be utilized.

Certainly, any one product for implementing the present invention is unnecessary to achieve all the above advantages at the same time.

### BRIEF DESCRIPTION OF THE DRAWINGS

Fig. 1 is a diagram of a gas path pipeline of a multichannel cryoablation system according to an embodiment of the invention;
Fig. 2 is a diagram of a channel pipeline and an electrical interface of an ablation needle of the multichannel cryoablation system according to an embodiment of the invention;
Fig. 3 is a hardware structure diagram of a control module of the multichannel cryoablation system according to an embodiment of the invention;
Fig. 4 is a flow chart of preparation for the ablation needle in a multichannel cryoablation control method according to an embodiment of the invention;
Fig. 5 is a reference diagram for the use of an ablation needle in the multichannel cryoablation control method according to an embodiment of the invention;
Fig. 6 is a flow chart of the control of electromagnetic valves in the multichannel cryoablation control method according to an embodiment of the invention.

### DESCRIPTION OF THE PREFERRED EMBODIMENTS

Although in the prior art, there are already multichannel cryoablation systems that use argon or cryogenic liquid nitrogen as a cold source, changes in the cold source will lead to changes in the design considerations of the entire device or system. Therefore, when a multichannel cryoablation device or system that employs the high-pressure nitrogen as the gas source is designed, it is not possible to simply and directly refer to the existing multichannel cryoablation system that employs argon or cryogenic liquid nitrogen as the cold source, instead it is necessary to re-establish an overall design theory based on the characteristics of the use of high-pressure nitrogen for the design and development of technical solutions.

The multichannel cryoablation system provided by the invention is a low-temperature cryotherapy system that employs the high-pressure nitrogen as the gas source, pre-cools the host, and then throttles flow to the cutter head. It has a high cooling capacity, and the gas source is cheaper and easier to obtain. In the multichannel cryoablation system of the invention, the high-pressure nitrogen is used as the gas source, which solves the problem of the use of nitrogen gas source when the available pressure range of the required gas source is different when more channels and fewer channels are used to complete the operation, solves the problem of isolation between channels and mutually exclusive access to a single resource due to not only the device but also shared structures individually owned by each of the channels when there are multiple ablation needle channels and each has functions such as freezing, rewarming and exhaust, and further solves the problem of identification and status judgment for the ablation needles of multiple channels as each of the channels may be inserted with the ablation needles when there are multiple channels.

The technical solutions of the disclosure will be described below clearly and comprehensively in conjunction with accompanying drawings. Apparently, the embodiments described are some embodiments rather than all embodiments of the disclosure. Based on the embodiments of the disclosure, all other embodiments obtained by those of ordinary skill in the art without creative efforts shall fall within the protection scope of the disclosure.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of the terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

### Embodiment

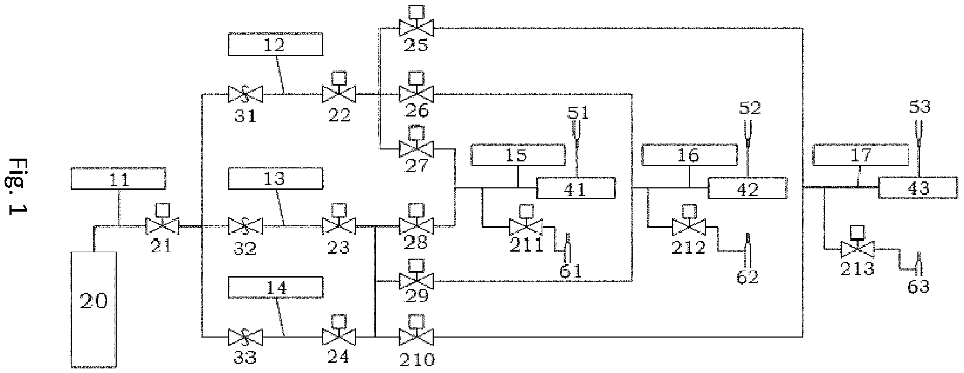

The gas pipeline of the multichannel cryoablation system of the invention includes a gas source input port used to connect a nitrogen cylinder, a plurality of pressure sensors, a plurality of electromagnetic valves, a plurality of pressure reducing valves, a plurality of pipe exhaust holes, a plurality of air outlets of the ablation needles, and a plurality of channel pipelines connected with the ablation needles. The gas pipeline will be described below in conjunction with Fig. 1, taking a three-channel cryoablation system as an example. On the premise that the invention discloses the three-channel cryoablation system, those skilled in the art can easily obtain the specific structure of the cryoablation system with other channels such as two channels, four channels, etc., and these modified embodiments are all within the protection scope of the invention.

With reference to Fig. 1, the gas pipeline of the multichannel cryoablation system of the embodiment is shown, wherein the nitrogen cylinder 20 is communicated with a main gas pipeline through the gas source input port, the pressure sensor 11 is used to obtain a gas pressure of the nitrogen cylinder 20, and the electromagnetic valve 21 is the main gas valve, which may control to realize the on/off of a high-pressure nitrogen in the main gas pipeline.

The main gas pipeline is subsequently communicated with a rewarming pipeline, a refrigeration high-pressure pipeline, and a refrigeration low-pressure pipeline, wherein
in the rewarming pipeline, the pressure reducing valve 31 is used to control a gas output pressure in the rewarming pipeline, the pressure sensor 12 is used to obtain an output pressure of the pressure reducing valve 31, and the electromagnetic valve 22 is a rewarming pipeline main valve, which may realize the on/off of the high-pressure nitrogen in the rewarming pipeline through control; the rewarming pipeline is divided into three rewarming branch channels following the electromagnetic valve 22, and the electromagnetic valves 25, 26, and 27 are respectively branch channel valves on the three rewarming branch channels of the rewarming pipeline; in other embodiments, there are several channels that correspond to several branch channel valves;
in the refrigeration high-pressure pipeline, the pressure reducing valve 32 is used to control an output pressure of the refrigeration high-pressure pipeline, the pressure sensor 13 is used to obtain an output pressure of the pressure reducing valve 32, and the electromagnetic valve 23 is a refrigeration high-pressure pipeline main valve, which may realize the on/off of the high-pressure nitrogen in the refrigeration high-pressure pipeline through control;
in the refrigeration low-pressure pipeline, the pressure reducing valve 33 is used to control an output pressure of the refrigeration low-pressure pipeline, the pressure sensor 14 is used to obtain an output pressure of the pressure reducing valve 33, and the electromagnetic valve 24 is a refrigeration low-pressure pipeline main valve, which may realize the on/off of the high-pressure nitrogen in the refrigeration low-pressure pipeline through control;
in Fig. 1, the refrigeration high-pressure pipeline and the refrigeration low-pressure pipeline are multiplexed, i.e., the refrigeration high-pressure pipeline and the refrigeration low-pressure pipeline share a section of pipeline following the electromagnetic valves 23 and 24; the above pipeline may be communicated with the three refrigeration branch channels, and the electromagnetic valves 28, 29 and 210 are respectively used as the branch channel valves on the three refrigeration branch channels.

The gas pipeline of the multichannel cryoablation system shown in Fig. 1 further includes three channel pipelines, and the three channel pipelines are pipelines connected with the ablation needles; the three channel pipelines are the same in structural configuration, wherein one end of each of the channel pipelines is divided into two branches, which are respectively connected with one of the channel valves of the rewarming branch channel of the rewarming pipeline and one of the channel valves of the refrigeration branch channel of the refrigeration pipeline (the refrigeration high-pressure pipeline and the refrigeration low-pressure pipeline are multiplexed); each of the channel pipelines is further connected with a branch pipe exhaust electromagnetic valve and the pipe exhaust hole, and each of the channel pipelines is further connected with the pressure sensor; the other end of each of the channel pipelines is connected with the ablation needle, and the ablation needle is connected with the air outlet or is provided with the air outlet. Specifically, the first channel pipeline has a left side connected with the electromagnetic valves 27, 28 and a right side is connected with the ablation needle 41, the ablation needle 41 is connected with an air outlet 51 or provided with the air outlet 51, and in addition, the first channel pipeline is further connected with a branch, which is provided with an electromagnetic valve 211 and a pipe exhaust hole 61 for pipe exhaust, the first channel pipeline being further connected with a pressure sensor 15; the second channel pipeline has a left side connected with the electromagnetic valves 26, 29 and a right side is connected with the ablation needle 42, the ablation needle 42 is connected with an air outlet 52 or provided with the air outlet 52, and in addition, the second channel pipeline is further connected with a branch, which is provided with an electromagnetic valve 212 and a pipe exhaust hole 62 for pipe exhaust, the first channel pipeline being further connected with a pressure sensor 16; the third channel pipeline has a left side connected with the electromagnetic valves 25, 210 and a right side is connected with the ablation needle 43, the ablation needle 43 is connected with an air outlet 53 or provided with the air outlet 53, and in addition, the third channel pipeline is further connected with a branch, which is provided with an electromagnetic valve 213 and a pipe exhaust hole 63 for pipe exhaust, the first channel pipeline being further connected with a pressure sensor 17.

If the high-pressure refrigeration of the first channel pipeline (connected to the ablation needle 41) is performed, the nitrogen flows from the nitrogen cylinder 20 through the electromagnetic valve 21, the pressure reducing valve 32, the electromagnetic valve 23, and the electromagnetic 28 to the ablation needle 41, and then is discharged through an outlet pipe of the ablation needle to the air outlet 51.

If the rewarming of the first channel pipeline (connected to the ablation needle 41) is performed, the nitrogen flows from the nitrogen cylinder 20 through the electromagnetic valve 21, the pressure reducing valve 31, the electromagnetic valve 22, and the electromagnetic valve 27 to the ablation needle 41, and then is discharged through an outlet pipe of the ablation needle to the air outlet 51.

If the high-pressure/low-pressure refrigeration and rewarming of the second/third channel pipeline are performed, a nitrogen flow path similar to the above may be obtained with reference to Fig. 1.

All the above pressure sensors are collectively referred to as a pressure sensor 1, and all electromagnetic valves are collectively referred to as an electromagnetic valve 2.

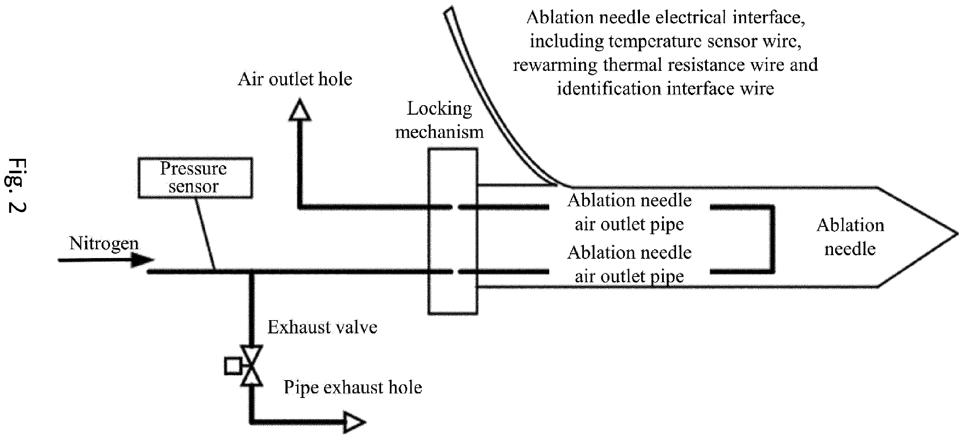

Fig. 2 shows a detailed diagram of the connection between one of the channel pipelines and the ablation needle, and the channel pipeline is connected with the pipeline (outlet pipe and inlet pipe) in the ablation needle through a locking mechanism. The ablation needle further includes an electrical interface, and the electrical interface may specifically include a temperature sensor wire, a rewarming thermal resistance wire, and an identification interface wire and so on.

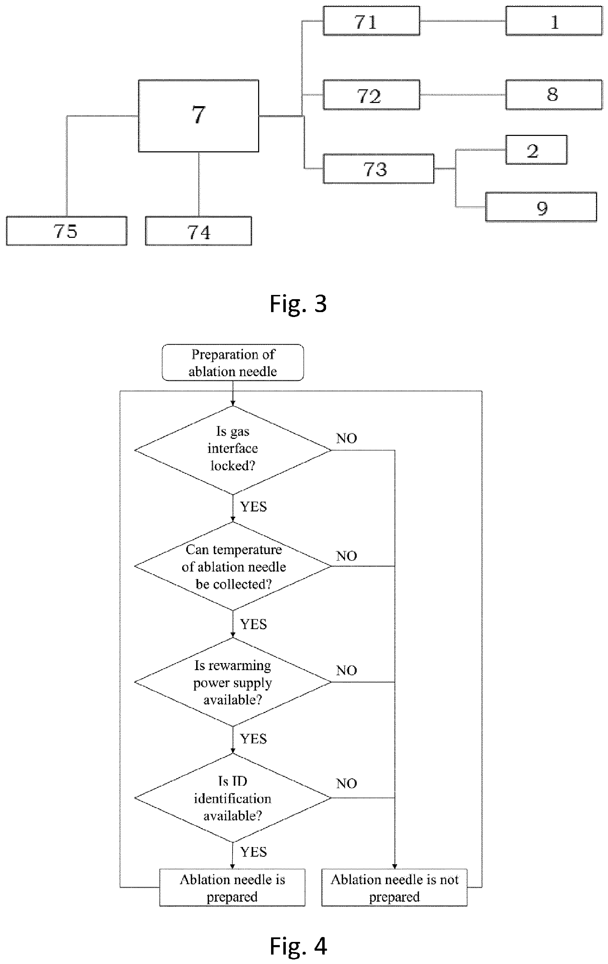

In the embodiment, the multichannel cryoablation system further includes a control circuit board; with reference to Fig. 3, taking the three-channel cryoablation system shown in Fig. 1 as an example, the control circuit board 7 obtains all the pressure data measured by all the pressure sensors 1 through a pressure measuring module 71, obtains all the temperature data of the ablation needle measured by the temperature sensor 8 through a temperature measuring module 72, controls the switch of the electromagnetic valve 2 and the rewarming power supply 9 of the ablation needle through a switch module 73, locks a gas interface between the ablation needle and the corresponding channel pipeline through an ablation needle locking module 74, and identifies each ablation needle through an ablation needle identification module 75. In the embodiment, all pressure reducing valves in Fig. 1 are manually adjusted, and in addition, all pipe exhaust holes and air outlets do not need to be controlled using a control module.

The following example illustrates the control method for cryoablation therapy using the above-mentioned multichannel cryoablation system, which is mainly divided into the following steps:
First, preparation of ablation needle:
Before the formal cryoablation, the ablation needle needs to be prepared, which means that the ablation needle is identified as usable by the control circuit board. Before the preparation of the ablation needles, the number of ablation needles to be used is firstly determined according to the volume of the tumor tissue.

In this step, the preparation of each of the ablation needles is independent, and the ablation needles of multiple channels will not interfere with each other during the preparation step.

This step requires the corresponding hardware as follows: for 3 ablation needles, 3 independent ablation needle identification interfaces are required, 3 independent ablation needle locking interfaces are required, 3 independent ablation needle rewarming power interfaces are required, and 3 independent temperature measuring interfaces are required. The ablation needle identification interface is used for a device to read and cancel ID information of the ablation needle, and the software in the control circuit board can use the ID information to identify the corresponding ablation needle; the ablation needle locking interface is a gas interface between the ablation needle and the corresponding device in the multichannel cryoablation system, wherein due to the high pressure of the gas flowing through the ablation needle, it is necessary to use a locking structure to lock the interface to prevent the ablation needle from being rushed out during ventilation; the rewarming power interface is an interface between the rewarming thermal resistance wire in the ablation needle and a rewarming power output wire in the device; the temperature measuring interface is an interface between the temperature sensor in the ablation needle and the temperature measuring module in the device.

As shown in Fig. 4, after the step of preparation of the ablation needle is performed, each of the ablation needles is marked as "prepared" or "unprepared" within the software. The process of preparation of ablation needle includes determining whether the gas interface is locked, whether the temperature of the ablation needle may be collected, whether the rewarming power supply is available, and whether the ID identification is available; after the above determinations, the control circuit board marks the ablation needle as "prepared" or "unprepared". The marking the ablation needle as "prepared" needs to meet the conditions that the gas interface is locked, the temperature of the ablation needle may be collected, the rewarming power supply is available, and the ID identification is available, and if any of the above conditions are not met, the ablation needle is marked as "unprepared". In Fig. 4, the order of determinations for the above conditions is only for illustration, and to realize the invention, it is not necessary to follow the order of determination shown in Fig. 4; as long as the determination of the above four conditions is completed, the marking results of the ablation needle may be obtained, and the order of determination of the four conditions is not limited in the invention.

During the operation stage, cryo-rewarming control needs to be completed according to the temperature of the ablation needle, so the temperature of the ablation needle should be collected when it is prepared. If the temperature sensor in the ablation needle fails, the temperature measuring module of the control circuit board may not collect the temperature of the ablation needle through the temperature measuring interface or the collected temperature value is abnormal, and then the ablation needle is marked as the "unprepared", which may effectively avoid subsequent operational risks.

In the rewarming stage, the software in the control circuit board turns on the rewarming power supply of the ablation needle, and the ablation needle is rewarmed. The ablation needle uses the rewarming thermal resistance wire to heat and rewarm, wherein the premise of completing this function is that the thermal resistance wire is intact and not broken. When the ablation needle is being prepared, the switch module of the control circuit board may detect the on/off of the thermal resistance wire of the ablation needle, thereby eliminating possible risks in advance.

The preparation of each of the ablation needles may refer to the above method steps, but not all ablation needles need to be prepared every time. The number of ablation needles that need to be prepared may be determined according to the volume of tumor tissue.

Second, selection of refrigeration high-pressure pipeline and refrigeration low-pressure pipeline:

The air pressure of the gas source is very important for the multichannel cryoablation system. Ideally, the higher the pressure of the nitrogen source, the better; however, limited by the capacity of the pipeline and the pressure of industrial nitrogen that may usually be purchased, the actual pressure of the gas source should be within a certain range.

Taking 15MPa (about 2200psi) industrial nitrogen as an example, after the nitrogen cylinder 20 is connected with the air inlet (also called the gas source input port) of the multichannel cryoablation system, the pressure sensor 11 may detect the cylinder pressure. With the use of nitrogen in the operation, the pressure of the gas cylinder will gradually decrease. When the pressure is lower than a lower limit, the ablation needle may not reach the operation temperature, and the operation may not continue.

For the multichannel cryoablation system of the invention, when different numbers of ablation needles are used simultaneously, the lower limit pressure values are different. The more a number of ablation needles are used simultaneously, the higher a lower limit pressure value becomes.

The refrigeration high-pressure pipeline uses the pressure reducing valve 32 to control the gas pressure in the pipeline, and the pressure reducing valve 32 is adjusted so that the output gas pressure is the lower limit pressure value 1;

The refrigeration low-pressure pipeline uses the pressure reducing valve 33 to control the gas pressure in the pipeline, and the pressure reducing valve 33 is adjusted so that the output gas pressure is the lower limit pressure value 2.

The relationship of the pressures can be seen from the above as *the lower limit pressure value 2 of the refrigeration low-pressure pipeline < the lower limit pressure value 1 of the refrigeration high-pressure pipeline < the gas cylinder pressure.*

Therefore, in the embodiment, when 3 ablation needles are used at the same time, the refrigeration high-pressure pipeline should be used; when less than 3 ablation needles are used at the same time, the refrigeration low-pressure pipeline should be used.

Thus, when the previous step of preparation of the ablation needle is completed and after the number of ablation needles to be used is determined depending on the volume of tumor tissue, the software of the control circuit board may judge the number of ablation needles prepared in the preparation stage of the ablation needle, and the refrigeration high-pressure pipeline or the refrigeration low-pressure pipeline is selected.

The rewarming pipeline uses the pressure reducing valve 31 to control the gas pressure in the pipeline, and the pressure reducing valve 31 is adjusted so that the output gas pressure is a lower limit pressure value 3, wherein the lower limit pressure value 3 is much lower than the lower limit pressure value 2. Normally, there is no insufficient rewarming gas pressure during the operation stage, and it can also be considered that the requirement for rewarming may be met if the gas cylinder pressure meets the requirement in refrigeration, so the selection of the rewarming pressure is not necessary.

Third, the use of ablation needles:
In the case of using three ablation needles at the same time, the complexity in control covers the situation of using one ablation needle alone. The following only uses three ablation needles at the same time as an example for illustration.

When three ablation needles are used at the same time, the function of each of the ablation needles corresponding to the channel is independent, and each channel has refrigeration, rewarming, and exhaust steps. As mentioned in the previous step, three ablation needles are used at the same time, and the refrigeration high-pressure pipeline should be used when in refrigeration.

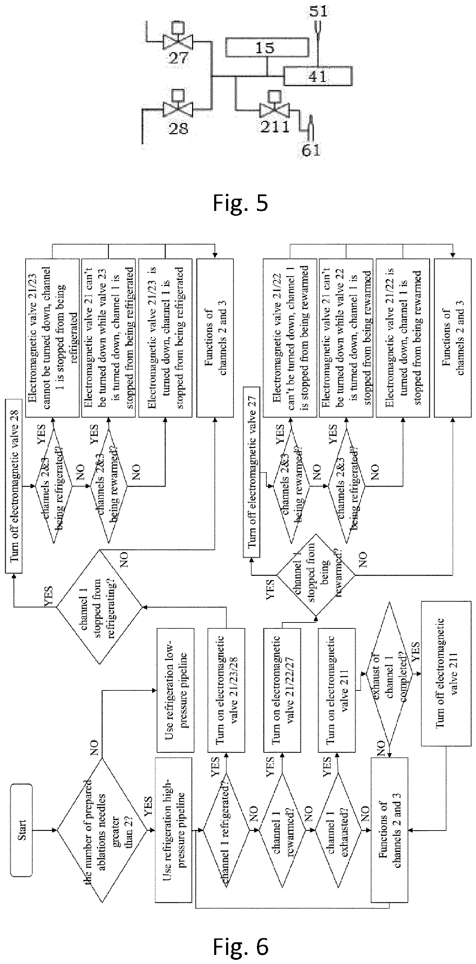

Taking the ablation needle 41 as an example, the process of refrigeration, rewarming, and exhaust will be described below with reference to Figs. 1 and 5.

Refrigeration of the ablation needle 41: the electromagnetic valves 21, 23, and 28 are initiated, and the nitrogen flows from the nitrogen cylinder 20 through the electromagnetic valve 21 to the pressure reducing valve 32 (the high-pressure pipeline), then to the electromagnetic valve 23, and then through the electromagnetic 28 to the ablation needle 41, and then is discharged through the outlet pipe of the ablation needle to the air outlet 51.

Rewarming of the ablation needle 41: the electromagnetic valves 21, 22, and 27 are initiated, and the nitrogen flows from the nitrogen cylinder 20 through the electromagnetic valve 21 to the pressure reducing valve 31, then to the electromagnetic valve 22, and then through the electromagnetic 27 to the ablation needle 41, and then is discharged through the outlet pipe of the ablation needle to the air outlet 51.

Exhaust of the ablation needle 41: the exhaust means exhausting the residual high-pressure gas in the channel pipeline, and during refrigeration and rewarming, the gas is discharged through the outlet pipe of the ablation needle, but the gas in the channel may not be exhausted; here, the channel pipeline refers to the pipeline intercepted by the electromagnetic valve 27, 28 and the air inlet pipe in the ablation needle 41, and this section of the pipeline is equipped with the pipeline exhaust hole 61, the electromagnetic valve 211, and the pressure sensor 15, as shown in Fig. 5. When the channel pipeline is exhausted, the electromagnetic valves 27 and 28 are closed, the electromagnetic valve 211 is opened, and the gas in the pipeline may be discharged through the exhaust hole 61 of the pipeline. Exhaust is an auxiliary function that may be used in various links. For example, the exhaust should be performed before unlocking the ablation needle gas interface. In addition, an exhaust operation may be performed immediately after the refrigeration or rewarming function has been completed.

As can be seen from the above, in the case of using three ablation needles, for the different functions of the gas channels of three ablation needle, the gas cylinder 20, the electromagnetic valve 21, the electromagnetic valve 22 and the electromagnetic valve 23 are shared; for the same gas channel of the ablation needle, the functions of refrigeration, rewarming and exhaust may not be used at the same time. The above restrictions increase the complexity in the control of each electromagnetic valve in the system, and the control method of each electromagnetic valve needs to ensure that the device may complete the expected function and is convenient and quick.

Fig. 6 shows a flow chart of controlling an electromagnetic valve. In Fig. 6, channel 1 refers to the channel of the ablation needle 41, channel 2 refers to the channel of the ablation needle 42, and channel 3 refers to the channel of the ablation needle 43, and "/" in Fig. 6 represents "and".

As shown in Fig. 6, if it is necessary to stop the refrigeration of channel 1, the electromagnetic valves 21, 23, and 28 may not be closed directly, and instead, it is necessary to confirm whether channels 2 and 3 are in refrigeration first, wherein if channels 2 and 3 are in refrigeration, it means that the electromagnetic valves 21 and 23 have also been opened, and then the only way to stop the refrigeration of channel 1 is to close the electromagnetic valve 28; if channels 2 and 3 are not in refrigeration, it is also necessary to confirm whether channels 2 and 3 are in rewarming, and if channels 2 and 3 are in rewarming, it means that the electromagnetic valve 21 is also opened, then the only way to stop the refrigeration of channel 1 is to close the electromagnetic valves 23, 28; if channels 2, 3 have neither in refrigeration nor rewarming, the way to stop the refrigeration of channel 1 may be closing the electromagnetic valves 21, 23, 28.

With reference to Fig. 5, if it is necessary to stop the rewarming of channel 1, the electromagnetic valves 21, 22, and 27 may not be closed directly, and instead, it is necessary to confirm whether channels 2 and 3 are in rewarming first, wherein if channels 2 and 3 are in rewarming, it means that the electromagnetic valves 21 and 22 have also been opened, and then the only way to stop the rewarming of channel 1 is to close the electromagnetic valve 27; if channels 2 and 3 are not in rewarming, it is also necessary to confirm whether channels 2 and 3 are in refrigeration, and if channels 2 and 3 are in refrigeration, it means that the electromagnetic valve 21 is also opened, then the only way to stop the rewarming of channel 1 is to close the electromagnetic valves 22, 27; if channels 2, 3 have neither in refrigeration nor rewarming, the way to stop the rewarming of channel 1 may be closing the electromagnetic valves 21, 22, 27.

The use of the exhaust function of the ablation needle will be described in detail below.

The functions of refrigeration and rewarming are the basic functions of cryoablation surgery, and the exhaust function is also described in the above steps of using the ablation needle. Exhaust is an auxiliary function that may be used in various links.

With reference to Fig. 5, the channel pipeline where the exhaust function needs to exhaust all the gas is a three-way channel pipeline, which is connected with the refrigeration pipeline and the rewarming pipeline of channel 1, and the air inlet pipeline of the ablation needle 41 at the same time. When there is residual high-pressure gas in the channel pipeline, the gas interface between the ablation needle and the device may not be unlocked, because the ablation needle may be flushed out after unlocking. Therefore, the exhaust should be performed before unlocking the ablation needle gas interface.

Further, the lower limit pressure values of the pressure reducing valve are different for the refrigeration function and the rewarming function, which means that the residual gas pressures in the channel pipeline are different; the residual gas pressure after the refrigeration function is completed is higher than the residual gas pressure after the rewarming is completed. If the rewarming function is initiated immediately after the refrigeration function is over, a failure will occur, i.e., the pressure at the output port of the pressure reducing valve 31 will be higher than the actual output pressure thereof for a short time, which is caused by the residual gas in the channel pipeline. Based on the above, an exhaust operation may be performed immediately after the refrigeration or rewarming function has been completed.

The control method of the embodiment also includes the use of the pressure sensor.

Specifically, the pressure sensor 11 is used to detect the pressure of the nitrogen cylinder, wherein when the pressure value of the nitrogen cylinder is lower than the lower limit pressure value 1 or the lower limit pressure value 2, the control method of the invention should prompt that the operation may not continue through the control circuit board.

The pressure sensors 12, 13, and 14 are used to detect the output pressure of the corresponding pressure reducing valve, wherein when the output pressure of the pressure reducing valve is manually adjusted, the output pressure value may be fed back to a regulator, so as to facilitate the adjustment of the output pressure of the pressure reducing valve. At the same time, after the adjustment of the pressure reducing valve is completed, the control circuit board may determine whether the output pressure value of the pressure reducing valve detected by the pressure sensor is within a predetermined range, and then determine the failure of the corresponding pressure reducing valve.

The pressure sensors 15, 16, and 17 are used to detect the pressure values of the corresponding channel pipelines. When the refrigeration and rewarming functions are performed, it can be judged whether the gas has reached here according to the detected pressure value. If the pipe is blocked, the gas may not reach here. Moreover, the exhaust process may also be determined according to the pressure values detected by the pressure sensors 15, 16, and 17, wherein when the exhaust function is performed, it can be known that the gas in the corresponding exhaust pipe has been exhausted when the pressure value detected by the pressure sensor is close to 0.

The control method described above may be implemented by software built into the control circuit board.

At last, it should be noted that the above various embodiments are only used to describe the technical solutions of the present invention, rather than limiting the technical solutions of the present invention. Even though the present invention is described in detail with reference to the foregoing embodiments, those of ordinary skilled in the art should understand that they can still modify the technical solutions recorded in the foregoing various embodiments or equivalently replace some or all of the technical features. However, these modifications or replacements do not make the essence of the corresponding technical solutions deviate from the scope of the technical solutions of the embodiments of the present invention. The present invention is merely limited by the appended claims and the scope and equivalents thereof.

## Claims

1. A multichannel cryoablation system, employing a high-pressure nitrogen as a gas source, the multichannel cryoablation system comprising:
a main gas pipeline provided with a gas source input port, a first pressure measuring device, and a main gas valve, wherein the gas source input port is used to be connected with a high-pressure nitrogen source, the first pressure measuring device is used to obtain a gas pressure of the high-pressure nitrogen cylinder, and the main gas valve may realize the on/off of the high-pressure nitrogen in the main gas pipeline;
a rewarming pipeline communicated with the main gas pipeline, the rewarming pipeline being provided with a first gas output pressure regulating device, a second pressure measuring device, and a rewarming pipeline main valve, wherein the first gas output pressure regulating device may regulate a gas output pressure of the rewarming pipeline, the second pressure measuring device is used to obtain an output pressure of the first gas output pressure regulating device, and the rewarming pipeline main valve may realize the on/off of the high-pressure nitrogen in the rewarming pipeline; the rewarming pipeline is divided into N rewarming branch channels following the rewarming pipeline main valve, and each of the rewarming branch channels is provided with a branch channel valve to realize the on/off of a gas circuit;
a refrigeration high-pressure pipeline and a refrigeration low-pressure pipeline, communicated with the main gas pipeline respectively, wherein the refrigeration high-pressure pipeline is provided with a second gas output pressure regulating device, a third pressure measuring device and a refrigeration high-pressure pipeline main valve; the second gas output pressure regulating device may regulate a gas output pressure of the refrigeration high-pressure pipeline, the third pressure measuring device is used to obtain an output pressure of the second gas output pressure regulating device, and the refrigeration high-pressure pipeline main valve may realize the on/off the high-pressure nitrogen in the refrigeration high-pressure pipeline; the refrigeration low-pressure pipeline is provided with a third gas output pressure regulating device, a fourth pressure measuring device and a refrigeration low-pressure pipeline main valve; the third gas output pressure regulating device may regulate a gas output pressure of the refrigeration low-pressure pipeline, the fourth pressure measuring device is used to obtain an output pressure of the third gas output pressure regulating device, and the refrigeration low-pressure pipeline main valve may realize the on/off of the high-pressure nitrogen in the refrigeration low-pressure pipeline; and a part of the refrigeration low-pressure pipeline following the refrigeration low-pressure pipeline main valve shares a section of gas path with a part of the refrigeration high-pressure pipeline following the refrigeration high-pressure pipeline main valve, and the gas path is communicated with the N refrigeration branch channels, each of the refrigeration branch channels being provided with a branch channel valve to realize the on/off of the gas circuit;
N channel pipelines, each of the channel pipelines having one end used to be connected with an ablation needle, and the other end used to be connected with one of the refrigeration branch channels and/or one of the rewarming branch channels; each of the channel pipelines is further provided with a fifth pressure measuring device, a pipe exhaust hole and a valve for controlling the opening and closing of the pipe exhaust hole; each of the ablation needles is connected with an air outlet or is provided with the air outlet;
wherein N is a positive integer greater than or equal to 2.

2. The multichannel cryoablation system according to claim 1, wherein N is a positive integer greater than or equal to 3.

3. The multichannel cryoablation system according to claim 1, wherein the main gas valve, the rewarming pipeline main valve, the refrigeration high-pressure pipeline main valve and the refrigeration low-pressure pipeline main valve are electromagnetic valves, the branch channel valve is the electromagnetic valve, and the valve for controlling the opening and closing of the pipe exhaust hole is the electromagnetic valve.

4. The multichannel cryoablation system according to claim 1, wherein the first/second/third gas output pressure regulating device is a pressure reducing valve.

5. The multichannel cryoablation system according to claim 3, wherein the ablation needle is further provided with an electrical interface, and the electrical interface comprises a temperature measurement device wire, a rewarming thermal resistance wire and an identification interface wire; the multichannel cryoablation system further comprises a control module, and the control module comprises a pressure measuring module, a temperature measuring module, a switch module, an ablation needle locking module and an ablation needle identification module, wherein the control module obtains all data of the pressure measuring device through the pressure measuring module, obtains all temperatures of the ablation needle through the temperature measuring module, controls switching of the electromagnetic valve and a rewarming power supply of the ablation needle through the switch module, locks a gas interface between the ablation needle and the channel pipeline through the ablation needle locking module, and identifies the ablation needle through the ablation needle identification module.

6. A multichannel cryoablation control method, capable of being implemented in the multichannel cryoablation system according to any one of claims 1 to 5, comprising:
preparation of the ablation needle: marking each of the ablation needles as "prepared" or "unprepared" in the control module, wherein the marking the ablation needle as "prepared" needs to meet the conditions that an ablation needle locking interface is locked, the temperature of the ablation needle may be collected, the rewarming power supply is available, and the ablation needle identification interface is available, and if any of the above conditions are not met, the correspondingly ablation needle is marked as "unprepared";
selection of the refrigeration high-pressure pipeline and the refrigeration low-pressure pipeline: determining to select the refrigeration high-pressure pipeline and the refrigeration low-pressure pipeline according to the number of the ablation needles prepared in the stage of the preparation of the ablation needle, wherein when more than three ablation needles are used, the refrigeration high-pressure pipeline is selected, and when less than three ablation needles are used, the refrigeration low-pressure pipeline is selected;
use of the ablation needle: cryoablating and rewarming the selected one or more ablation needles, wherein when the on/off of the gas path is controlled at some stage in the process of cryoablating and rewarming each of the ablation needles, the opening and closing is required to be performed for the main gas pipeline, the rewarming pipeline main valve, the refrigeration high-pressure pipeline main valve or the refrigeration low-pressure pipeline main valve according to requirements for the gas path at the stage of treatment using the remaining ablation needles of the shared pipeline.

7. The multichannel cryoablation control method according to claim 6, further comprising an exhaust method of exhausting the gas before unlocking an ablation needle gas interface.

8. The multichannel cryoablation control method according to claim 6, wherein further comprising performing one of the exhausting operations after a refrigerating function or a rewarming function of the ablation needle is completed.

9. The multichannel cryoablation control method according to claim 6, wherein further comprising sending a prompt signal when the first pressure measuring device detects that a pressure value of the gas cylinder is lower than a preset lower limit pressure value.

10. The multichannel cryoablation control method according to claim 6, wherein an output pressure of the first/second/third gas output pressure regulating device is regulated manually, the control module determines whether the output pressure of the first/second/third gas output pressure regulating device is within a predetermined range according to the pressure value when the first/second/third gas output pressure regulating device is regulated to further judge failures of the first/second/third gas output pressure regulating device.

11. The multichannel cryoablation control method according to claim 6, further comprising determining whether the gas reaches the place, by the control module, according to a pressure value measured by the fifth pressure measuring device on each of the channel pipelines.

12. The multichannel cryoablation control method according to claim 6, further comprising determining that a gas in an exhaust pipe on the channel pipeline has been exhausted when an exhausting function of the ablation needle is performed such as the control module receiving information that the pressure value measured by the fifth pressure measuring device on the channel pipeline is close to 0.

13. A multichannel cryoablation system, employing a high-pressure nitrogen as a gas source, comprising:
a main gas pipeline, provided with a gas source input port, the gas source input port being used to be connected with the high-pressure nitrogen source; the main gas pipeline is connected and communicated with at least one rewarming pipeline and at least one refrigeration pipeline respectively, wherein
each of the rewarming pipelines is provided with a first gas output pressure regulating device, and the first gas output pressure regulating device may regulate a gas output pressure of the corresponding rewarding pipeline; each of the rewarming pipelines is divided into N rewarming branch channels following the first gas output pressure regulating device, each of the rewarming branch channels is provided with a branch channel valve, and the branch channel valve may realize the on/off of the high-pressure nitrogen in the rewarming branch channel;
each of the refrigeration pipelines is provided with a second gas output pressure regulating device, and the second gas output pressure regulating device may regulate a gas output pressure of the refrigeration pipeline; each of the refrigeration pipelines is divided into N refrigeration branch channels following the second gas output pressure regulating device, each of the refrigeration branch channels is provided with a branch channel valve, and the branch channel valve may realize the on/off of the high-pressure nitrogen in the refrigeration branch channel;
N channel pipelines, each of the channel pipelines having one end used to be connected with an ablation needle, and the other end used to be connected with one of the refrigeration branch channels and/or one of the rewarming branch channels;
wherein N is a positive integer greater than or equal to 2.

14. The multichannel cryoablation system according to claim 13, comprising more than two refrigeration pipelines, wherein pressures in the refrigeration pipelines are different from each other.

15. The multichannel cryoablation system according to claim 14, wherein the more than two refrigeration pipelines are designed as a multiplexing structure, at least two refrigeration pipelines share a section pipeline following the respective second gas output pressure regulating device, and the shared section of pipeline may be communicated with each of the refrigeration branch channels.

16. The multichannel cryoablation system according to claim 13, wherein each of the rewarming pipelines is provided with a rewarming pipeline main valve, and the rewarming pipeline main valve may realize the on/off of the high-pressure nitrogen in the rewarming pipeline; each of the refrigeration pipelines is provided with a refrigeration pipeline main valve, and the refrigeration pipeline main valve may realize the on/off of the high-pressure nitrogen in the refrigeration pipeline.

17. The multichannel cryoablation system according to claim 13 or 16, wherein the main gas pipeline is provided with a main gas valve, and the main gas valve may realize the on/off of the high-pressure nitrogen in the main gas pipeline.

18. The multichannel cryoablation system according to claim 13, further comprising the first pressure measuring device provided on the main gas pipeline, the first pressure measuring device is used to measure a gas pressure of the high-pressure nitrogen cylinder.

19. The multichannel cryoablation system according to claim 13 or 18, wherein each of the rewarming pipelines is provided with a second pressure measuring device, and the second pressure measuring device is used to obtain an output pressure of the first gas output pressure regulating device; each of the refrigeration pipelines is provided with a third pressure measuring device, and the third pressure measuring device is used to obtain an output pressure of the second gas output pressure regulating device.

20. The multichannel cryoablation system according to claim 13, wherein each of the channel pipelines is further provided with a pressure measuring device.

21. The multichannel cryoablation system according to claim 13, wherein each of the channel pipelines is further provided with a pipe exhaust hole, and a valve for controlling the opening and closing of the pipe exhaust hole; each of the ablation needles is connected with an air outlet or is provided with the air outlet.

22. The multichannel cryoablation system according to claim 18 or 19, wherein all valves used in the pipelines to perform the on-off are electromagnetic valves.

23. The multichannel cryoablation system according to claim 13, wherein the first/second gas output pressure regulating device is a pressure reducing valve.

24. The multichannel cryoablation system according to claim 22, wherein the ablation needle is provided with an electrical interface, and the electrical interface comprises a temperature measurement device wire, a rewarming thermal resistance wire and an identification interface wire; the multichannel cryoablation system further comprises a control module, and the control module comprises a pressure measuring module, a temperature measuring module, a switch module, an ablation needle locking module and an ablation needle identification module, wherein the control module obtains all data of the pressure measuring device through the pressure measuring module, obtains all temperatures of the ablation needle through the temperature measuring module, controls switching of the electromagnetic valve and a rewarming power supply of the ablation needle through the switch module, locks a gas interface between the ablation needle and the channel pipeline through the ablation needle locking module, and identifies the ablation needle through the ablation needle identification module.
